# EUROPÄISCHE PATENTSCHRIFT

(11) **EP 1 714 692 B1**
(45) Veröffentlichungstag und Bekanntmachung des Hinweises auf die Patenterteilung: **09.03.2011**
(21) Anmeldenummer: 05008649.5
(22) Anmeldetag: 20.04.2005
(51) Int. Cl.: B01D 63/02

(54) **Dialysefilter**
Dialysis filter
Filtre de dialyse

(43) Veröffentlichungstag der Anmeldung: 25.10.2006
(73) Patentinhaber: B. Braun Avitum AG, 49215 Glandorf (DE)
(72) Erfinder: Heinrich, Bernd, Dr., 01833 Dürröhrsdorf-Dittersbach (DE); Martins, Konrad, Dr., 01309 Dresden (DE); Mehnert, Dieter, 01454 Radeberg (DE); Müller, Eckard, 01454 Radeberg (DE); Kracht, Katrin, 01279 Dresden (DE); Franke, Horst, 01189 Dresden (DE); Hector, Rainer, Dr., 49082 Osnabrück (DE)
(74) Vertreter: Klingseisen, Franz

(56) Entgegenhaltungen:
- EP-A- 0 514 021
- EP-A- 0 562 520
- EP-A- 0 798 034
- EP-A- 1 088 792
- EP-A- 1 092 468
- WO-A-03/029744
- WO-A-2004/050221
- DE-A1- 3 711 695
- US-A- 4 141 835
- US-A- 4 681 720
- US-A- 4 876 066
- US-A- 5 450 516
- US-A- 5 489 413
- US-A- 5 598 874

## Beschreibung

Die Erfindung betrifft eine Filtervorrichtung nach dem Oberbegriff des Anspruchs 1 und insbesondere einen Dialysefilter.

Ein Dialysefilter dieser Art ist aus US-A- 5 489 413 bekannt, bei dem das Hohlfaserbündel ringförmig in einem Gehäuse um einen mittigen Hohlraum angeordnet ist.

EP-A-0 798 034 beschreibt eine Hohlfaserfiltervorrichtung, bei der die Hohlfasern um einen mittigen, langgestreckten Dorn angeordnet sind, der Perforationen für den Durchtritt des Filtermediums aufweist. Die Hohlfasern sind schraubenlinienförmig gewunden in einem Gehäuse angeordnet und an den Enden in einer Vergussmasse gehalten.

Aufgabe der Erfindung ist es, eine Filtervorrichtung der eingangs angegebenen Art so auszubilden, dass die Filterleistung verbessert wird.

Nach der Erfindung wird eine Verbesserung der Filterleistung dadurch erreicht, dass die Packungsdichte des Hohlfaserbündels über die Länge des Bündels variiert und das Hohlfaserbündel insbesondere im Ein- und/oder Ausströmbereich des Dialysats aufgeweitet ist, während die Packungsdichte im Mittelbereich des Hohlfaserbündels höher ist. Hierdurch wird ein möglichst ungehinderter radialer Fluss des Dialysats im aufgeweiteten Bereich des Bündels erreicht, sodass sich eine gleichmäßige Strömungsverteilung über den gesamten Bündelquerschnitt ergibt. Während aufgrund der geringeren Packungsdichte im Ein- und ggfs. auch im Ausströmbereich der radialen Strömung ein geringer Strömungswiderstand entgegengesetzt wird, wird im Hauptteil des Filters durch die höhere Packungsdichte der Hohlfasern eine gleichmäßige Umströmung der einzelnen Hohlfasern in Längsrichtung gewährleistet.Hierdurch wird ein optimaler Stoffabtransport vor allem auch in den Ein- und Ausströmbereichen und damit eine Verbesserung der Filterleistung erzielt.

Die Erfindung wird beispielsweise anhand der Zeichnung näher erläutert. Es zeigen
- Fig. 1: einen schematischen Längsschnitt durch einen Dialysefilter,
- Fig. 2: eine Draufsicht auf die Schnittfläche des Hohlfaserbündels mit Dichtung,
- Fig. 3: einen Querschnitt durch ein Verteilerelement und eine Draufsicht,
- Fig. 4: Ablenkelemente im Einströmbereich des Fluids zwischen die Hohlfasern, und
- Fig. 5: schematisch Varianten der Wellung der Hohlfasern im Bündel.

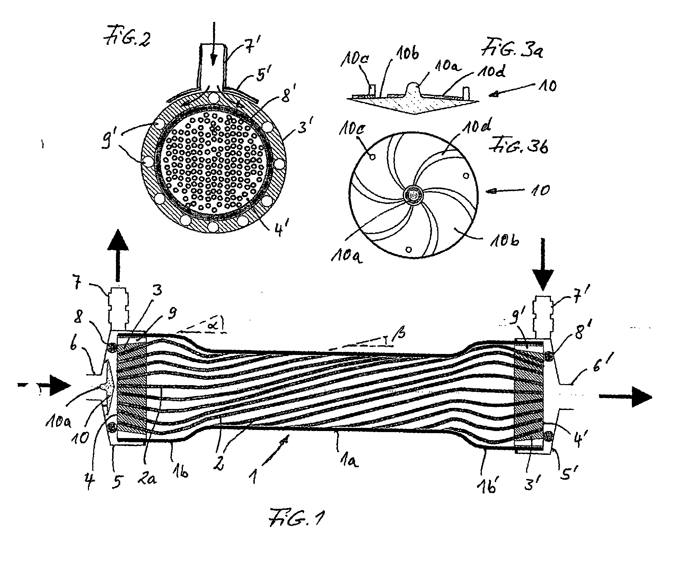

Fig. 1 zeigt in einem Längsschnitt einen Dialysator mit einem allgemein rohrförmigen Gehäuse 1, das an den Stirnseiten durch Kappen 5 und 5' geschlossen ist. In dem Gehäuse 1 ist ein Bündel aus semi-permeablen Hohlfasern 2 durch zwei scheibenförmige Blöcke 3 und 3' einer Vergussmasse positioniert, deren Umfang auf dem Innenumfang des rohrförmigen Gehäuses 1 anliegt und in die die Enden der einzelnen Hohlfasern 2 des Bündels fluiddicht eingebettet sind. Die jeweilige Außenseite der Vergussmasse 3 bzw. 3' bildet eine ebene Schnittfläche 4 bzw. 4' der Hohlfasern quer zur Längsachse des Gehäuses mit Öffnung zum Innern der Hohlfasern.

Bei dem dargestellten Ausführungsbeispiel in Fig. 1 liegt die Schnittebene 4 im Wesentlichen auf der Höhe des Randes des Gehäuses 1, wobei sich die Kappen 5 über den Endbereich des Gehäuses 1 erstrecken und gegenüber dem Gehäuse abgedichtet sind. Die Schnittebene 4 kann aber auch versetzt zum Rand des Gehäuses angeordnet sein.

Die Kappen 5 und 5' am Gehäuse 1 sind jeweils mittig mit einer Einlassleitung 6 bzw. einer Auslassleitung 6' versehen, die im Wesentlichen längs der Längsache des rohrförmigen Gehäuses 1 verläuft. Bei einem Dialysator wird durch die Leitung 6 Blut zugeführt und über die Leitung 6' abgeführt. Dialysat wird im Gegenstrom durch eine radial in die Kappe 5 mündende Leitung 7' zugeführt und durch eine Leitung 7 abgeführt. Die Strömungsrichtung ist durch Pfeile in Fig. 1 wiedergegeben.

Mit 8 und 8' ist ein Dichtungsring zwischen Kappe 5 und Schnittfläche 4 bezeichnet, der zwei Fluidkammern im Dialysator voneinander trennt, eine erste Fluidkammer, die die Einlassleitung 6 und die Auslassleitung 6' mit dem zentralen Bereich in den Kappen 5 und 5' sowie das Innere der Hohlfasern 2 umfasst, und eine zweite Fluidkammer, die die Leitungen 7 und 7' für das Dialysat mit dem Strömungsbereich außerhalb des Dichtungsringes 8 in den Kappen und den Innenraum des Gehäuses 1 zwischen den einzelnen Hohlfasern 2 umfasst.

Außerhalb des Dichtungsringes 8' sind in der Vergussmasse 3' im Wesentlichen axial verlaufende Kanäle 9' vorzugsweise ungleichmäßig über den Umfang verteilt angeordnet, wie Fig. 2 zeigt, durch die das über die Einlassleitung 7' einströmende Dialysat in die zweite Fluidkammer zwischen den Hohlfasern eingeleitet wird. In gleicher Weise sind im Austrittsbereich des Dialysats Fluidkanäle 9 außerhalb des Dichtungsringes 8 auf dem Umfang der Vergussmasse 3 verteilt angeordnet.

Die im Wesentlichen axial verlaufenden Kanäle 9', die in Fig. 2 mit einem runden Querschnitt dargestellt sind, aber auch einem langgestreckten Rechteckquerschnitt haben können, haben im Einlaufbereich vorzugsweise eine sich in Strömungsrichtung verjüngende Form, um die Strömungsverteilung im Einlaufbereich zu unterstützen. Auf der Austrittsseite sind die Kanäle 9 aus herstellungstechnischen Gründen in gleicher Weise ausgebildet. In diesem Bereich können sie auch eine durchgehend gleiche Querschnittsform haben.

Die auf dem Umfang der Vergussmasse 3' vorgesehenen Kanäle 9' können in ihrem Abstand und/oder Querschnitt unterschiedlich gestaltet sein, um das Strömungsprofil insbesondere im Einlaufbereich zu beeinflussen. Fig. 2 zeigt durch einen Pfeil das durch die Einlassleitung 7' in radialer Richtung einströmende Dialysat. Damit nicht ein Hauptteil des Dialysats durch die Kanäle 9' nahe der Einlassleitung 7' in die Fluidkammer zwischen den Hohlfasern einströmt, während an den diametral gegenüberliegenden Kanälen 9' weniger Dialysat zuströmen würde, sind die Kanäle 9' auf der der Einlassleitung 7' gegenüberliegenden Seite dichter längs des Umfangs angeordnet als im Bereich der Einlassleitung 7'. Hierdurch wird die Zuströmung des Dialysats in die Fluidkammer um die Hohlfasern gleichmäßiger über den Umfang verteilt.

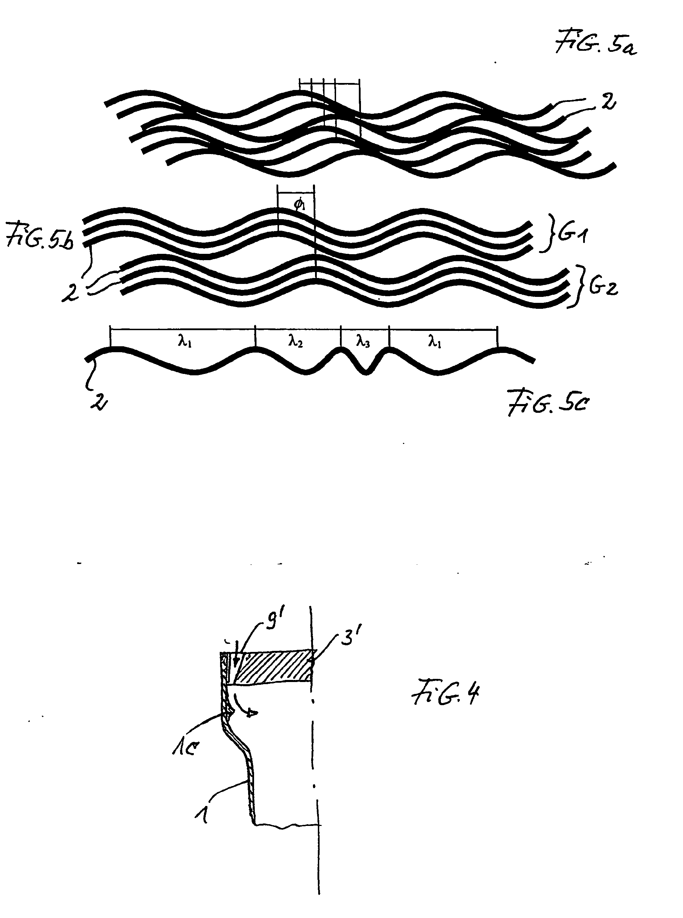

Fig. 4 zeigt im Einströmbereich des Dialysats stromab von den Kanälen 9' ein Ablenkelement an der Innenwand des Gehäuses 1 in Form eines über den Umfang umlaufenden oder nur nahe den einzelnen Kanälen bzw. Durchbrüchen 9' ausgebildeten Absatzes 1 c, durch den das einströmende Dialysat radial nach innen gelenkt wird, wie dies durch einen Pfeil angedeutet ist. Dieses Ablenkelement 1c kann als Rippe am Gehäuse angeformt sein.

In der mit der Einlassleitung 6 versehenen Kappe 5 ist ein scheibenförmiges Verteilerelement 10 mittig quer zur Längsachse angeordnet. Das durch die Einlassleitung 6 zuströmende Fluid trifft etwa auf die Mitte des Verteilerelementes 10, sodass die Strömung von der Mitte aus radial nach außen gleichmäßig verteilt wird. Der Außenumfang des Verteilerelementes 10, der einen Abstand von dem Dichtungsring 8 hat, wird hierdurch gleichmäßig umströmt. Zur Verbesserung der Verteilung der Strömung zum Umfang hin ist das Verteilerelement 10 auf der Seite des durch die Leitung 6 zuströmenden Fluids mit einer zapfenförmigen Erhöhung 10a in der Mitte versehen, die eine abgerundete Stirnfläche aufweist. Von der zapfenförmigen Erhöhung 10a erstreckt sich eine Verteilerfläche 10b zum Umfangsrand des Verteilerelementes 10. Bei dem Ausführungsbeispiel nach Fig. 3 erstreckt sich die Verteilerfläche von der zapfenförmigen Erhöhung 10a zum Umfangsrand etwa in einem Winkel von 90° zur Längsachse. Die Verteilerfläche kann auch flach-kegelförmig von der Mitte nach außen verlaufen, sodass der Winkel auf der Zuströmseite zwischen Verteilerfläche 10b und Längsachse größer als 90° ist. Bei einer solchen flach-kegelförmigen Gestaltung kann die zapfenförmige Erhöhung 10a auch entfallen, wobei sie durch die Spitze der Kegelform ersetzt wird.

Bei einer solchen Ausgestaltung in Verbindung mit der in Fig. 1 und 3 wiedergegebenen flachen Kegelform auf der Unterseite der Verteilerscheibe ergibt sich ein insgesamt etwa diskusförmiges Verteilerelement 10. Die Verteilerfläche 10b kann auch leicht konkav gewölbt ausgebildet sein, sodass sie sich von einer mittigen Erhöhung entsprechend 10a in einer Bogenlinie zum Umfang erstreckt.

Bei 10c sind schematisch über den Umfang verteilte Abstandhalter (beispielsweise drei) wiedergegeben, durch die das Verteilerelement 10 an der Stirnseite der Kappe 5 gehalten wird. Das scheibenförmige Verteilerelement 10 kann auch durch Erhebungen auf der Ober- und/oder Unterseite oder auch durch speichenartige Elemente auf dem Umfang in der Kappe 5 in seiner Lage festgelegt sein, wobei diese stellenweise vorgesehenen Erhöhungen oder Speichen im Querschnitt strömungsgünstig gestaltet sind.

Wie Fig. 3b zeigt, kann die der Einlassleitung 6 zugewandte Seite des Verteilerelementes 10 vorteilhafterweise auch mit spiralförmig gestalteten Rippen 10d oder Vertiefungen versehen sein, um dem zuströmenden Fluid einen Drall zu erteilen. In gleicher Weise kann auf der der Schnittfläche 4 zugewandten Seite eine die Strömungsverteilung begünstigende Strukturierung vorgesehen werden, zusätzlich zu der flach-kegelförmigen Gestalt oder auch anstelle von dieser.

Auf der der Schnittfläche 4 zugewandten Seite hat das Verteilerelement 10 bei dem dargestellten Ausführungsbeispiel in Fig. 1 eine flach-kegelförmige Gestalt, wobei die Spitze einen geringen Abstand von der Schnittfläche 4 hat, sodass die Strömung über die Schnittfläche 4 von außen nach innen gleichmäßig verteilt und die Zuströmung zu den einzelnen Hohlfasern 2 radial innen durch die kleiner werdende Spaltdicke begünstigt wird.

Die Strömungsgeschwindigkeit zwischen Verteilerelement 10 und Schnittfläche 4 kann auch dadurch beeinflusst bzw. gleichmäßig gestaltet werden, dass die Oberfläche der Schnittfläche 4 nicht eben, sondern flach-kegelförmig ausgebildet wird.

Bei dem Ausführungsbeispiel nach Fig. 1 ist die Wand der Kappe 5 so geformt, dass sich eine zum Umfang hin kleiner werdende Spaltdicke zwischen Verteilerfläche 10b und Innenfläche der Kappe 5 ergibt, um die Geschwindigkeitsverteilung gleichmäßig zu gestalten. Dabei ist die Spaltform vorzugsweise so gestaltet, dass die Strömungsgeschwindigkeit v des zuströmenden Fluids radial nach außen langsamer abnimmt als entsprechend der Formel v = const./r, die sich bei einer konstanten Spaltdicke ergeben würde.

Die Spaltdicke kann also durch die Kappenform und/oder die Formgebung der Verteilerscheibe 10 radial nach außen kleiner werdend ausgebildet werden.

Das Hohlfaserbündel 2 hat eine über seine Länge unterschiedliche Packungsdichte. Insbesondere ist im Zulaufbereich bei 1b die Packungsdichte geringer als - in axialer Richtung gesehen - im Mittelbereich des Hohlfaserbündels. Die Packungsdichte ist in Prozent ausgedrückt der Anteil der Bündelquerschnittsfläche, der durch Hohlfasern ausgefüllt wird. Im Falle runder Hohlfaserquerschnitte ist die theoretisch maximal erreichbare Packungsdichte 90,7 %. Praktisch realisierte Packungsdichten liegen im Bereich von 40 bis 60 %. Vorzugsweise liegt die Packungsdichte im Zulaufbereich bei etwa 30 % bis 40 %, vorzugsweise bei 35 % und im Mittelbereich des Hohlfaserbündels bei etwa 45 bis 55 %, bevorzugt 50 %.

Im aufgeweiteten Bereich hat das Hohlfaserbündel eine Packungsdichte, die wenigstens um 5, vorzugsweise 10 kleiner ist als die in Prozent angegebene Packungsdichte im Mittelbereich, sodass beispielsweise bei einer Packungsdichte im Mittelbereich von 50 % die Packungsdichte im aufgeweiteten Bereich bei 40 %, höchstens bei 45 % liegt.

Durch die geringere Packungsdichte im Zulaufbereich des Dialysats ergibt sich eine verbesserte radiale Strömung zwischen die Hohlfasern. Hierdurch wird erreicht, dass das Dialysat bereits im Einlaufbereich vollständig mit den Filterflächen der Hohlfasern in Kontakt kommt und dadurch die Länge der Hohlfasern hinsichtlich Filterwirkung voll ausgenutzt wird. Im anschließend dichteren Bereich ergibt sich eine Strömung in Längsrichtung bei gleichmäßiger Umströmung der einzelnen Fasern ohne Kanalbildung zwischen den Hohlfasern.

Wie Fig. 1 zeigt, sind die in die Vergussmasse 3 eingebetteten Enden der Hohlfasern 2 so angeordnet, dass sich innerhalb der Vergussmasse 3 in Richtung auf die Schnittfläche 4 eine größere Packungsdichte ergibt als im Bereich niedrigster Packungsdichte angrenzend an die Innenseite der Vergussmasse 3. Mit anderen Worten verlaufen vor allem die radial äußeren Hohlfasern im Bereich der Vergussmasse 3 schräg zur Längsachse des Dialysefilters, sodass sie an der Schnittfläche 4 innerhalb des Dichtungsringes 8 dichter gepackt sind und auf der gegenüberliegenden Seite der Vergussmasse 3 sich bis nahe an den Außenumfang der Vergussmasse 3 aufweiten. Der Bündeldurchmesser ist somit an der Schnittfläche 4 kleiner und vergrößert sich bereits im Bereich der Vergussmasse.

In Fig. 1 ist ein Winkel α wiedergegeben, der den Neigungswinkel beispielsweise der radial äußeren Hohlfaser zur Längsachse wiedergibt, wobei diese Hohlfaser in Richtung der Längsachse verläuft, also in einer um 90° verdrehten Ansicht auf der Längsachse liegt.

Eine höhere Packungsdichte insbesondere im Mittelbereich des Hohlfaserbündels 2 kann auch durch eine unterschiedlich starke Verdrillung der Hohlfasern über ihre Länge erreicht werden. Hierbei wird die an sich runde Querschnittsform der Fasern zu einer ovalen Schnitt- bzw. Projektionsfläche senkrecht zur Längsachse. Fig. 1 zeigt - in Längsrichtung gesehen - im Mittelbereich des Hohlfaserbündels 2 eine Verdrillung der Hohlfasern um die Längsachse. Mit anderen Worten verlaufen die einzelnen Hohlfasern im Endbereich im Wesentlichen unverdrillt auf die Vergussmasse zu, wobei sie im Wesentlichen längs der Längsachse verlaufen, während sie im Mittelbereich verdrillt oder stärker verdrillt sind, wobei sie einen Winkel β zur Längsachse bilden. Deutlich wird dies beispielsweise bei der schematischen Darstellung in Fig. 1 an der im Einströmbereich in der Mitte liegenden Hohlfaser 2a, die im Einlaufbereich im Wesentlichen sich längs der Längsachse erstreckt, worauf sie im Mittelbereich einen schrägen Verlauf zur Längsachse einnimmt und sich um diese herum erstreckt, sodass sie im Umfangsbereich der Vergussmasse 3' unter der Zeichenebene mündet. Im Auslaufbereich hat diese Hohlfaser einen radialen Abstand von der Längsachse, wobei sie im Wesentlichen in deren Richtung verläuft mit einer Neigung zur Längsachse hin.

Nach einer vorteilhaften Ausgestaltung wird das Gehäuse 1 so gestaltet, dass es sich an die unterschiedliche Packungsdichte des Hohlfaserbündels 2 anpasst. Das Gehäuse 1 ist deshalb im Mittelbereich 1a in Längsrichtung gesehen eingeschnürt, sodass es das Hohlfaserbündel 2 im dichteren Packungsbereich umschließt, während sich das Gehäuse 1 in den Endbereichen 1b und 1b' glocken- oder kegelförmig nach außen erweitert. Dabei kann in den Endbereichen 1b und 1b' mit größerem Durchmesser die Innenwand des Gehäuses einen radialen Abstand von den äußeren Hohlfasern haben, wie dies auch Fig. 1 zeigt.

Vorzugsweise ist auch der Austrittsbereich des Hohlfaserbündels 2 mit einer geringeren Pakkungsdichte versehen entsprechend der Ausgestaltung im Einlaufbereich. Auch hierdurch wird eine verbesserte radiale Strömung zu den Austrittsöffnungen bzw. Kanälen 9' in der Vergussmasse 3' erreicht.

Bevorzugt liegen die Hohlfasern in allen Bereichen der variierenden Packungsdichte an der Gehäusewand stellenweise an, wobei ein derartiges vollständiges Ausfüllen des Gehäusequerschnitts im Bereich unterschiedlicher Gehäusedurchmesser durch die mechanische Eigenspannungen gewellter Hohlfasern erreicht werden kann. Durch die mechanische Eigenspannung von gewellten Hohlfasern spreizen sich diese auseinander und liegen an der Innenwand des Gehäuses an.

Die Erweiterung des Hohlfaserbündels 2 wird zweckmäßigerweise glockenförmig oder kegelförmig gestaltet, damit das Dialysat zunächst radial und dann in Achsrichtung längs der Hohlfasern über deren Länge strömt.

Zur Verbesserung des Kontakts der Dialysatströmung mit den Hohlfasern sind diese über ihre Länge gewellt und vorzugsweise insgesamt zumindest im Mittelbereich 1a des Gehäuses verdrillt angeordnet, wie dies Fig. 1 schematisch zeigt. Die Wellenform der einzelnen Hohlfasern bzw. Gruppen von Hohlfasern ist in Fig. 5 schematisch wiedergegeben, wobei verschiedene Formen der Wellung gezeigt sind. Durch die Wellung der Hohlfasern wird einerseits erreicht, dass die Hohlfasern nur an einzelnen Stellen aneinanderliegen und damit ihre maximale Oberfläche für die Filterwirkung zur Verfügung steht, und andererseits, dass durch Verwirbelung der Umströmung der Hohlfasern der Stoffaustausch verbessert wird. Zudem verhindert die Faserwellung auch die Kanalbildung zwischen den Hohlfasern, d. h. Bereiche zu geringer Packungsdichte, durch die das Dialysat bevorzugt strömt, ohne zur Filterleistung beizutragen.

Fig. 5a zeigt einzelne Hohlfasern 2 mit etwa gleicher Wellenlänge, wobei die Wellungen in Längsrichtung versetzt zueinander angeordnet sind entsprechend einer Verschiebung der Phasenlage.

Fig. 5b zeigt zwei Gruppen G₁ und G₂ mit jeweils drei gewellten Hohlfasern, wobei die Hohlfasern innerhalb einer Gruppe G in der gleichen Phasenlage verlaufen, während die benachbarte Gruppe eine mit Φ₁ bezeichnete Versetzung der Phasenlage aufweist. Vorzugsweise bilden 2 bis 20, vorzugsweise 8, Hohlfasern eine Gruppe. Zumindest in einer bestimmten Menge von Fasergruppen G weist eine Gruppe eine vorgegebene Versetzung der Phasenlage zur benachbarten Gruppe auf. Hierdurch ergibt sich eine geordnete Struktur der Anordnung der gewellten Hohlfasern innerhalb eines Bündels. Zwischen parallel zueinanderliegenden Hohlfasern oder auch bei gleicher Phasenlage nebeneinanderliegender gewellter Hohlfasern, wie dies innerhalb einer Gruppe G der Fall ist, verläuft die Strömung des Dialysats längs der Hohlfasern, ohne dass dieses Strömungsmuster verändert wird. Durch die versetzte Wellung benachbarter Hohlfasern oder benachbarter Hohlfasergruppen G wird dieses Strömungsmuster unterbrochen bzw. tritt eine Verwirbelung der Strömung des Dialysats um die Hohlfasern auf, wodurch die Filterleistung verbessert wird.

Bei der Herstellung eines Hohlfaserbündels wird bevorzugt eine vorgegebene Menge von Gruppen G mit einer vorgegebenen Versetzung Φ₁ der Wellung relativ zueinander ausgebildet, wobei das gesamte Bündel aus mehreren solchen Mengen zusammengesetzt wird. Auf diese Weise wird erreicht, dass zumindest im Bereich einer Menge die einzelnen Fasergruppen die vorgegebene Versetzung der Phasenlage haben und damit eine optimale Umströmung der Gruppen von Hohlfasern erreicht wird.

Fig. 5c zeigt eine einzelne Hohlfaser 2 mit über ihre Länge variierender Wellung, wobei verschiedene Wellenlängen λ1 bis λ3 wiedergegeben sind.

Nach einer anderen Ausgestaltung kann sich die Wellenlänge der Faserwellung periodisch mit einer Periode verändern, die mindestens doppelt so groß ist wie die größte Wellenlänge.

## Patentansprüche

1. Filtervorrichtung, insbesondere zur Entfernung von Substanzen aus Blut oder anderen Körperflüssigkeiten, umfassend
- ein Bündel aus semi-permeablen Hohlfasern (2),
- die an ihren Enden in einer Vergussmasse (3) eingebettet und gehalten sind, und
- ein rohrförmiges Gehäuse (1), das das Hohlfaserbündel umgibt,
- wobei Ein- und Auslassleitungen (6, 7; 6', 7') an den Gehäuseenden mit den in dem Gehäuse ausgebildeten getrennten Fluidräumen verbunden sind,
wobei das Hohlfaserbündel (2) eine über seine Länge derart variierende Packungsdichte aufweist, dass das Hohlfaserbündel zumindest im Einströmbereich des die Hohlfasern umgebenden Fluidraumes eine niedrigere Packungsdichte aufweist als im anschließenden Mittelbereich,
**dadurch gekennzeichnet,**
**daß** im Bereich der in die Vergussmasse (3) eingebetteten Faserenden die Packungsdichte höher ist als in den Bereichen niedrigster Packungsdichte angrenzend an die Vergussmasse.

2. Filtervorrichtung nach Anspruch 1, wobei im Ausströmbereich des die Hohlfasern umgebenden Fluidraumes im Wesentlichen die gleiche Packungsdichte vorgesehen ist wie im Einströmbereich.

3. Filtervorrichtung nach Anspruch 1 oder 2, wobei das Hohlfaserbündel in den Bereichen geringerer Packungsdichte kegel- oder glockenförmig aufgeweitet ist.

4. Filtervorrichtung nach einem der vorhergehenden Ansprüche, wobei die in Längsrichtung des Hohlfaserbündels variierende Packungsdichte mindestens zum Teil durch eine unterschiedlich starke Verdrillung der Hohlfasern (2) in den verschiedenen Bereichen erreicht wird.

5. Filtervorrichtung nach einem der vorhergehenden Ansprüche, wobei die Hohlfasern eine wellenförmige Struktur haben und in den Endbereichen und im Mittelbereich über die Länge des Bündels an der Gehäusewand mindestens stellenweise anliegen.

## Claims

1. Filter device, in particular for removing substances from blood or other body fluids, comprising
- a bundle of semi-permeable hollow fibers (2),
- the ends of which are embedded and held in a casting compound (3), and
- a tubular casing (1) surrounding the hollow fiber bundle,
- wherein at the ends of the casing, inlet and outlet pipes (6, 7; 6', 7') are connected with the partitioned fluid chambers formed in the casing,
wherein the hollow fiber bundle (2) has a packing density which is varied over its length such that, at least in the inflow portion of the fluid chamber surrounding the hollow fibers, the hollow fiber bundle has a lower packing density than in the adjacent middle portion,
**characterized in that**
in the area of the fiber ends embedded in the casting compound (3), the packing density is higher than in the areas of lowest packing density abutting on the casting compound.

2. Filter device according to claim 1, wherein in the outflow portion of the fluid chamber surrounding the hollow fibers, substantially the same packing density is provided as in the inflow portion.

3. Filter device according to claim 1 or 2, wherein the hollow fiber bundle is widened in a cone- or bell-shaped manner in the areas of lower packing density.

4. Filter device according to one of the preceding claims, wherein the packing density varying in the longitudinal direction of the hollow fiber bundle is achieved at least partly by a different degree of twist of the hollow fibers (2) in the different portions.

5. Filter device according to one of the preceding claims, wherein the hollow fibers have an undulated structure and in the end portions and in the middle portion they abut at least in places at the casing wall over the length of the bundle.

## Revendications

1. Un dispositif de filtrage en particulier pour enlever des substances dans du sang ou dans des autres liquides organiques, comprenant
- une botte de fibres (2) creuses et semi-perméables,
- dont les bouts sont enfoncés et tenus dans une masse de scellement,
- un boîtier (1) tubulaire qui entoure la botte de fibres creuses et semi-perméables,
- dans lequel aux bouts du boîtier, des tuyaux d'entrée et de sortie (6, 7; 6', 7') sont raccordés aux chambres de fluide cloisonnées formées dans le boîtier,
dans lequel la botte de fibres (2) creuses et semi-perméables a une densité de tassement qui varie sur toute sa longueur de façon qu'au moins dans la zone d'arrivée de la chambre de fluide entourant les fibres creuses, la botte de fibres creuses et semi-perméables a une moindre densité de tassement que dans la zone centrale avoisinante, **caractérisé en ce que**
dans la zone des extrémités des fibres enfoncées dans la masse de scellement (3), la densité de tassement est plus haute que dans les zones ayant la densité de tassement la plus basse qui aboutissent à la masse de scellement.

2. Un dispositif de filtrage selon la revendication 1, dans lequel dans la zone de la sortie de la chambre de fluide entourant les fibres creuses, on prévoit substantiellement la même densité de tassement comme dans la zone d'entrée.

3. Un dispositif de filtrage selon l'une des revendications 1 ou 2, dans lequel la botte de fibres creuses s'élargit en forme conique ou en forme de cloche dans les zones ayant une moindre densité de tassement.

4. Un dispositif de filtrage selon l'une quelconque des revendications précédentes, dans lequel la densité de tassement de la botte des fibres creuses varie dans la direction longitudinale et est obtenue au moins en partie par un degré différent de torsion des fibres creuses dans les zones différentes.

5. Un dispositif de filtrage selon l'une quelconque des revendications précédentes, dans lequel les fibres creuses ont une structure ondulée et aboutissent au moins en partie à la paroi du boîtier dans-les zones des extrémités et dans la zone centrale sur toute la longueur de la botte.
